# EUROPÄISCHE PATENTSCHRIFT

(11) **EP 3 457 986 B1**
(45) Veröffentlichungstag und Bekanntmachung des Hinweises auf die Patenterteilung: **29.07.2020**
(21) Anmeldenummer: 17723992.8
(22) Anmeldetag: 15.05.2017
(51) Int. Cl.: A61F 2/14, B29D 11/02, B29D 11/00

(54) **OPHTHALMOLOGISCHES IMPLANTAT**
OPHTHALMOLOGICAL IMPLANT
IMPLANT OPHTHALMOLOGIQUE

(30) Priorität: 17.05.2016 DE 102016208395
(43) Veröffentlichungstag der Anmeldung: 27.03.2019
(73) Patentinhaber: HumanOptics AG, 91054 Erlangen (DE)
(72) Erfinder: MESSNER, Arthur, 91220 Schnaittach (DE)
(74) Vertreter: Rau, Schneck & Hübner Patentanwälte Rechtsanwälte PartGmbB
(86) Internationale Anmeldenummer: PCT/EP2017/061628
(87) Internationale Veröffentlichungsnummer: WO 2017/198627

(56) Entgegenhaltungen:
- CN-B- 102 090 941
- DE-A1- 19 850 807
- DE-A1-102007 042 637
- US-A- 3 679 504
- US-A1- 2008 304 009
- US-B1- 6 221 106

## Beschreibung

Die Erfindung betrifft ein ophthalmologisches Implantat gemäß dem Oberbegriff des Anspruchs 1, das oftmals auch als Irisimplantat bezeichnet wird. Ferner richtet sich die Erfindung auf ein Verfahren zur Herstellung einer Pigmentanordnung als Bestandteil eines solchen ophthalmologischen Implantats.

Zum Ersatz einer teilweise oder vollständig fehlenden Iris werden heutzutage üblicherweise in der Peripherie gefärbte Kontaktlinsen oder ophthalmologische Implantate verwendet. Ein ophthalmologisches Implantat ist beispielsweise aus der DE 10 2007 042 637 A1 bekannt. Dieses hat sich in der Praxis bewährt.

Bekanntlich werden ophthalmologische Implantate in einem oder in verschiedenen Außendurchmessern hergestellt. Zur Anpassung des zu implantierenden ophthalmologischen Implantats an individuelle anatomische Verhältnisse eines zu behandelnden Auges bzw. einer zu behandelnden teilweisen Aniridie wird im Allgemeinen das ophthalmologische Implantat in seinem Außendurchmesser verkleinert. Dies kann beispielsweise mit einem Trepan, einer Schere oder einem Skalpell erfolgen. Nachteilig ist, dass dadurch im Allgemeinen die Biokompatibilität des ophthalmologischen Implantats beeinträchtigt wird.

Aus der DE 198 50 807 A1 ist ein gattungsgemäßes künstliches Irissystem bekannt, das als ophthalmologisches Implantat ausgeführt sein kann. Das Irissystem umfasst mindestens zwei bioverträgliche Folien, die verschiedene Farben haben und übereinander angeordnet sind. Die obere Folie hat Pigmentflecken. Hinter den Folien ist eine schwarze Folie angeordnet. Die Folien sind kreisringförmig. Zum Erhalten einer glatten Oberfläche sind die Folien mit einem durchsichtigen bioverträglichen Material umhüllt. Die Biokompatibilität dieses Implantats ist oftmals unbefriedigend.

Aus der US 2008/0304009 A1 sind gefärbte Kontaktlinsen bekannt, die abwechselnd klare Lagen und Farblagen haben.

Die CN 102090941 B offenbart eine künstliche Iris mit Farbänderungsvermögen.

Die US 3,679,504 offenbart ein Verfahren zum Bilden von Farbeffekten in Kontaktlinsen und ophthalmologischen Implantaten. Farbmuster werden unter Verwendung beispielsweise von Pigmenten gebildet.

Eine aus der US 6,221,106 B1 bekannte Membran zur Implantation in einen Kapselsack umfasst einen Fixierring, der gefärbtes PMMA aufweist.

Der Erfindung liegt daher die Aufgabe zugrunde, ein ophthalmologisches Implantat zu schaffen, das die Nachteile des Standes der Technik überwindet. Insbesondere soll ein ophthalmologisches Implantat geliefert werden, das auch nach seiner Anpassung an die individuellen anatomischen Verhältnisse des zu behandelnden Auges bzw. der zu behandelnden teilweisen Aniridie eine äußerst hohe Biokompatibilität aufweist. Ein entsprechendes Verfahren zur Herstellung von Pigmentanordnungen als Bestandteil eines solchen ophthalmologischen Implantats soll ebenfalls bereitgestellt werden.

Diese Aufgabe wird erfindungsgemäß durch die in dem Anspruch 1 und 16 angegebenen Merkmale gelöst. Der Kern der Erfindung liegt darin, dass das mindestens eine Farbpigment der Pigmentanordnung zumindest größtenteils von Umhüllungsmaterial umhüllt ist. Hierdurch ist das mindestens eine Farbpigment der Pigmentanordnung insbesondere abgeschottet bzw. gekapselt, was die Biokompatibilität des ophthalmologischen Implantats, insbesondere auch nach dessen Anpassung, verbessert.

Das ophthalmologische Implantat weist eine besonders hohe Biokompatibilität auf. Dies gilt insbesondere dann, wenn jedes Farbpigment vollständig umhüllt ist. Selbst bei einer Anpassung des ophthalmologischen Implantats an die individuellen anatomischen Verhältnisse des zu behandelnden Auges ist diese besonders hohe Biokompatibilität gegeben.

Günstigerweise hat mindestens eine, bevorzugter mehrere, bevorzugter die meisten, bevorzugter alle, der Pigmentanordnungen mindestens ein umhülltes Farbpigment.

Es ist von Vorteil, wenn die mindestens eine, eine Umhüllung aufweisende Pigmentanordnung geringfügig in der Grundstruktur verlagerbar ist. Die Umhüllung bleibt dann insbesondere auch bei einer Anpassung des ophthalmologischen Implantats unbeschädigt.

Das mindestens eine Farbpigment ist günstigerweise lichtdicht. Vorzugsweise ist dieses lichtempfindlich. Das mindestens eine Farbpigment ist beispielsweise aus photosensiblen Substanzen, wie Pyrane, Oxazine und/oder Fulgide, hergestellt. Es kann aber auch durch ein Metalloxid oder nichtmetallische feste Stoffe gebildet sein. Günstigerweise ist eine Vielzahl von Farbpigmenten mit unterschiedliche Farben vorhanden. Insbesondere ähnelt das ophthalmologische Implantat einer natürlichen Iris, was eine ästhetische Rehabilitation erlaubt.

Günstigerweise hat das mindestens eine Farbpigment, bevorzugter mehrere, bevorzugter die meisten, bevorzugter alle, der Farbpigmente, ein Spannungs-Dehnungs-Verhalten, das sich von einem Spannungs-DehnungsVerhalten der Umhüllung und/oder der Grundstruktur, insbesondere stark, unterscheidet. Beispielsweise liegt ein Unterschied in dem Elastizitätsmodul, der Zugfestigkeit und/oder der Verformbarkeit vor. Insbesondere hat das mindestens eine Farbpigment eine Steifigkeit bzw. einen Widerstand gegen elastische Verformung, die/der mindestens 50 % größer, bevorzugter mindestens 100 % größer, als die Steifigkeit bzw. der Widerstand gegen elastische Verformung der Umhüllung und/oder der Grundstruktur ist.

Die Grundstruktur hat insbesondere einen veränderlichen Transmissionsgrad, wobei vorzugsweise der Transmissionsgrad der Grundstruktur bei Bestrahlung des ophthalmologischen Implantats mit Licht mit zunehmender Intensität des Lichts abnimmt.

Es ist von Vorteil, wenn die Grundstruktur kreisringförmig ist. Sie hat günstigerweise einen Außendurchmesser, der zur Anpassung an die individuellen anatomischen Verhältnisse des zu behandelnden Auges reduzierbar ist. Es ist zweckmäßig, wenn das ophthalmologische Implantat zur Implantation elastisch verformbar ist.

Die zentrale Apertur ist vorzugsweise kreisförmig. Sie ist insbesondere flüssigkeitsdurchlässig ausgebildet. Es ist zweckmäßig, wenn die zentrale Apertur lichtdurchlässig ist und insbesondere einen hohen Transmissionsgrad aufweist. Der Transmissionsgrad ist insbesondere größer als 0,9.

Die Umhüllung ist günstigerweise dünnwandig. Sie ist aus einem Umhüllungsmaterial gebildet. Es ist von Vorteil, wenn zwischen dem Umhüllungsmaterial und dem Grundstrukturmaterial eine chemische und/oder physikalische Verbindung vorliegt.

Weitere vorteilhafte Ausgestaltungen der Erfindung sind in den Unteransprüchen angegeben.

Das mindestens eine Farbpigment gemäß dem Unteranspruch 2 ist äußerst einfach herstellbar. Ferner hat ein solches ophthalmologisches Implantat eine äußerst hohe Biokompatibilität, selbst nach Anpassung dessen Außenabmessung.

Günstigerweise hat die Umhüllung gemäß dem Unteranspruch 4 eine Umhüllungsdicke, die wesentlich kleiner als eine Abmessung, insbesondere Höhe, Breite, Länge und/oder Dicke, des mindestens einen Farbpigments der jeweiligen Pigmentanordnung ist.

Bei der Umhüllungsdicke gemäß dem Unteranspruch 5 treten aus Fertigungsgründen oftmals Abweichungen auf.

Das biokompatible Material gemäß dem Unteranspruch 6 ist/hat günstigerweise ein Polymermaterial, wie, insbesondere hochreines, Silikon, Acrylat und/oder Methacrylat. Es ist zweckmäßig, wenn die Umhüllung aus dem biokompatiblen Material vollständig gebildet ist. Das biokompatible Material ist vorzugsweise in mindestens einer Monoschicht der Umhüllung angeordnet. Es bildet dann das Umhüllungsmaterial.

Die reaktiven Monomere gemäß dem Unteranspruch 7 sind vorzugsweise in mindestens einer Reaktionsschicht der Umhüllung angeordnet. Das ophthalmologische Implantat erhält diese vorzugsweise während seiner Herstellung.

Es ist von Vorteil, wenn die Fäden gemäß dem Unteranspruch 8 mit einer Matrix, die insbesondere durch das biokompatible Material gebildet ist, verschlungen sind. Günstigerweise bilden die Fäden eine äußere Schicht der Umhüllung. Die Fäden sind vorzugsweise in der Umhüllung ganz außen angeordnet. Sie sind vorzugsweise aus einem biokompatiblen Material, wie Silikon, Acrylat und/oder Methacrylat, gebildet. Günstigerweise haben die Fäden jeweils eine Länge von mindestens 25 µm. Es ist von Vorteil, wenn die Dicke der Fäden jeweils kleiner als 5 µm ist. Es ist von Vorteil, wenn das Längen-/Durchmesserverhältnis der Fäden zwischen 3:1 und 7:1 liegt. Günstigerweise liegt eine physikalische Verbindung zwischen Umhüllung und dem Grundstrukturmaterial vor.

Gemäß dem Unteranspruch 9 hat jede Pigmentanordnung einen Umkreisdurchmesser, der kleiner als 30 µm, bevorzugter kleiner als 10 µm, ist. Die Gestalt des mindestens einen Farbpigments der jeweiligen Pigmentanordnung ist günstigerweise beliebig.

Das ophthalmologische Implantat gemäß dem Unteranspruch 11 ist günstigerweise im Bereich der bereichsweise überlappend angeordneten Pigmentanordnungen im Wesentlichen in einer Blickrichtung des ophthalmologischen Implantats lichtdicht.

Gemäß dem Unteranspruch 12 sind die Grundstruktur durch ein Grundstrukturmaterial und die Umhüllung durch ein Umhüllungsmaterial gebildet, wobei sich das Grundstrukturmaterial und das Umhüllungsmaterial voneinander unterscheiden. Beispielsweise unterscheiden sich das Grundstrukturmaterial und das Umhüllungsmaterial in ihrer Zusammensetzung, Dichte, Lichtdurchlässigkeit und/oder Lichtabsorption.

Die Ausgestaltung gemäß dem Unteranspruch 13 führt zu einer besonders hohen Biokompatibilität des ophthalmologischen Implantats. Das Umhüllungsmaterial ist günstigerweise härter, insbesondere wesentlich härter, als das Grundstrukturmaterial. Es hat somit insbesondere einen höheren mechanischen Widerstand gegen ein mechanisches Eindringen eines anderen Körpers als das Grundstrukturmaterial. Diese größere Härte ist beispielsweise durch einen höheren Vernetzungsgrad, insbesondere Quervernetzungsgrad, und/oder eine andere Materialzusammensetzung erreicht. Das mindestens eine Farbpigment ist günstigerweise härter, insbesondere wesentlich härter, als das Grundstrukturmaterial. Es hat somit insbesondere einen höheren mechanischen Widerstand gegen ein mechanisches Eindringen eines anderen Körpers als das Grundstrukturmaterial.

Nachdem insbesondere das mindestens eine Farbpigment inklusive die zugehörige Umhüllung härter, insbesondere wesentlich härter, als die Grundstruktur bzw. das Grundstrukturmaterial ist, ist so sichergestellt, dass auch bei einer Anpassung des ophthalmologischen Implantats an die individuellen anatomischen Verhältnisse eines zu behandelnden Auges diese Pigmentanordnung innerhalb der Grundstruktur einem Anpassungs- bzw. Verkleinerungswerkzeug, wie einer Schere, einem Trepan oder Skalpell, ausweicht und die Umhüllung unversehrt bleibt.

Alternativ ist beispielsweise nur die Umhüllung härter als die Grundstruktur. Das mindestens eine Farbpigment hat eine geringere Härte als die Grundstruktur. Alternativ entspricht die Härte des mindestens einen Farbpigments der Härte der Grundstruktur.
Die Gegenstände der Ansprüche 2 bis 15 können auch bevorzugte Weiterbildungen des Anspruchs 16 betreffen.

Nachfolgend wird unter Bezugnahme auf die beigefügte Zeichnung eine bevorzugte Ausführungsform der Erfindung beispielhaft beschrieben. Dabei zeigen:
- Fig. 1: eine Vorderansicht eines erfindungsgemäßen ophthalmologischen Implantats,
- Fig. 2: einen Längsschnitt durch das in Fig. 1 gezeigte ophthalmologische Implantat entsprechend der Schnittlinie II-II,

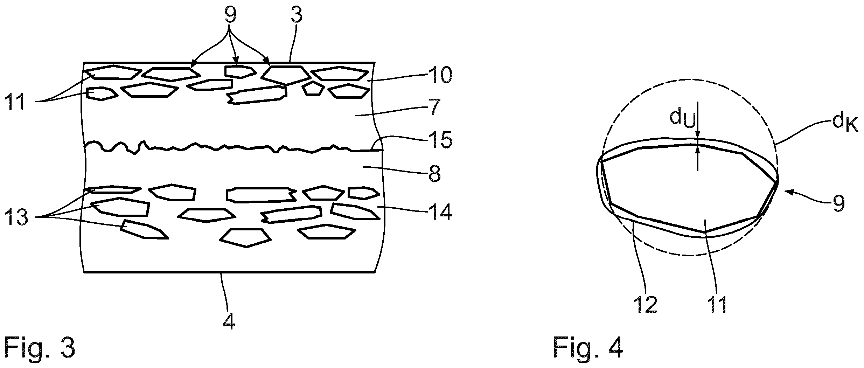

- Fig. 3: einen vergrößerten Teilschnitt des in den Fig. 1 und 2 gezeigten ophthalmologischen Implantats, und
- Fig. 4: eine vergrößerte Ansicht einer Pigmentanordnung des in den Fig. 1 und 2 dargestellten ophthalmologischen Implantats.

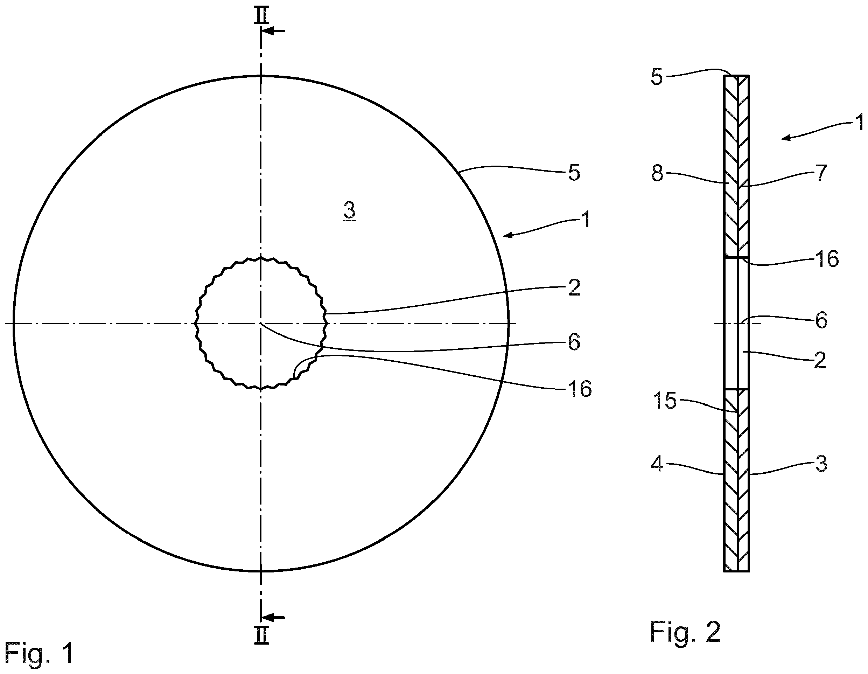

Ein in den Fig. 1 und 2 in der Gesamtheit dargestelltes ophthalmologisches Implantat, das insbesondere eine natürliche Iris nachbildet, umfasst eine ringförmige Grundstruktur 1 mit einer zentralen, im Wesentlichen kreisförmigen Apertur 2.

Die Grundstruktur 1 ist im Wesentlichen flächig ausgebildet und hat eine Vorderseite 3 und eine der Vorderseite 3 gegenüberliegende Rückseite 4. Die Vorderseite 3 ist günstigerweise plan bzw. glatt ausgebildet. Es ist von Vorteil, wenn die Grundstruktur 1 auf der Vorderseite 3 eine strukturierte Oberfläche aufweist, welche der einer natürlichen Iris nachempfunden ist. Es ist von Vorteil, wenn die Rückseite 4 insbesondere plan bzw. glatt ist. Alternativ weist die Vorderseite 3 und/oder Rückseite 4 eine leicht konvexe Umhüllende auf.

Umfangsseitig ist die Grundstruktur 1 außen von einer Randfläche 5 begrenzt, die um eine Mittelachse 6 der Grundstruktur 1 läuft.

Die Grundstruktur 1 hat einen äußeren Durchmesser, der mindestens 10 mm, insbesondere mindestens 12 mm, beträgt. Der äußere Durchmesser ist beispielsweise durch Trepanieren verkleinerbar. Hierbei wird mittels eines scharfen Hohlstempels, dessen Innendurchmesser dem gewünschten äußeren Durchmesser des ophthalmologischen Implantats entspricht, ein überstehender Kreisring abgeschert. Dadurch ist die Größe des ophthalmologischen Implantats flexibel an die jeweils gewünschte Größe anpassbar.

Die Grundstruktur 1 hat in Richtung der Mittelachse 6 eine Dicke, die zwischen 0,1 mm und 0,4 mm liegt.

Die Apertur 2 hat einen Durchmesser im Bereich von 1 mm bis 8 mm, insbesondere von 3 mm bis 5 mm. Sie ist insbesondere als freie Öffnung ausgebildet.

Die Apertur 2 wird umfangseitig durch eine innere Randfläche 16 begrenzt. Die innere Randfläche 16 ist insbesondere rau ausgebildet und weist kleine Einkerbungen und Fortsätze auf, die abwechselnd angeordnet sind.

Die Grundstruktur 1 ist mehrlagig, hier zweilagig. Sie umfasst eine vorderseitige Lage 7 und eine rückseitige Lage 8. Die Lagen 7, 8 sind längs der Mittelsachse 6 derart angeordnet, dass die vorderseitige Lage 7 die Vorderseite 3 und die rückseitige Lage 8 die Rückseite 4 aufweist bzw. bildet.

Die vorderseitige Lage 7 ist vorzugsweise aus Silikon gebildet. Die rückseitige Lage 8 ist vorzugsweise aus Silikon gebildet. Andere polymerisarbare Stoffe sind alternativ möglich.

In die vorderseitige Lage 7 ist eine Vielzahl von Pigmentanordnungen 9 eingebettet. Die vorderseitige Lage 7 ist insgesamt vorzugsweise zumindest teilweise lichtundurchlässig. Die Pigmentanordnungen 9 sind in der vorderseitigen Lage 7 günstigerweise in einer äußeren Schicht 10 der vorderseitigen Lage 7, also benachbart zu der Vorderseite 3, angeordnet. Sie überlappen sich bereichsweise in Richtung der Mittelachse 6 und/oder senkrecht zu dieser. Die Pigmentanordnungen 9 sind unterschiedlich farbig ausgeführt.

Jede Pigmentanordnung 9 hat ein inneres Farbpigment 11 und eine dieses Farbpigment 11 vollständig umhüllende, äußere Umhüllung 12. Die Pigmentanordnungen 9 haben jeweils einen Umkreisdurchmesser d_{K}, der kleiner als 30 µm, bevorzugter kleiner als 10 µm, ist.

Die Umhüllungen 12 haben vorzugsweise eine im Wesentlichen einheitliche Umhüllungsdicke du. Die Umhüllungen 12 sind vollständig aus einem biokompatiblen Polymermaterial, wie Silikon, Acrylat und/oder Methacrylat, gebildet. Günstigerweise sind die Umhüllungen 12 und die Grundstruktur 1, insbesondere deren vorderseitige Lage 7, grundsätzlich aus einem identischen Material hergestellt. Vorzugsweise ist zu deren Bildung Zwei-Komponenten-Silikon eingesetzt.

Die Umhüllungen 12 haben günstigerweise zumindest außenseitig reaktive Monomere, die im Stande sind, mit umgebenden Monomeren zu reagieren und eine chemische Bindung einzugehen.

Die Umhüllungen 12 sind beispielsweise gemäß einer alternativen, bevorzugten Ausführungsform zumindest außenseitig fadenförmig ausgeführt. Die Fäden sind mit einer umgebenden Matrix aus dem biokompatiblen Material der jeweiligen Umhüllung 12 verschlungen, was zu einer festen mechanischen und/oder physikalischen Verbindung führt. Eine Ausgestaltung ohne Fäden ist möglich.

Die Pigmentanordnungen 9 werden beispielsweise hergestellt, indem reine Farbpigmente 11 in geringer Konzentration in hochreiniges, flüssiges Biomaterial dispergiert werden. Die Konzentration der Farbpigmente 11 wird anschließend durch Filtration des flüssigen Biomaterials erhöht. Die dann umhüllten Farbpigmente 11 hoher Konzentration werden unter Bedingungen aufbewahrt, die eine Weiterverarbeitung über den Aufbewahrungszeitraum sicherstellen.

Die rückseitige Lage 8 enthält Pigmente 13, die in einer Schicht 14 angeordnet sind. Die Pigmente 13 sind im Stande, sichtbares Licht zu absorbieren. Sie sind günstigerweise schwarz. Alternativ entsprechen die Pigmente 13 den Farbpigmenten 11.

Die Lagen 7, 8 unterscheiden sich insbesondere durch die Konzentration und/oder die Art der verwendeten Pigmente 11, 13.

Zwischen den Lagen 7, 8 liegt eine Grenzfläche 15 vor, die wellig oder glatt ausgeführt ist. Bei welliger Ausbildung haben die Wellen günstigerweise eine Höhe, die größer als 0,025 mm, bevorzugter größer als 0,05 mm, bevorzugter größer als 0,1 mm, ist.

Die Grenzfläche 15 kann Bestandteil eines Grenzbereichs sein, in welcher ein gradueller Übergang in Bezug auf Konzentration und/oder Art der verwendeten Pigmente 11, 13 zwischen der vorderseitigen Lage 7 und der rückseitigen Lage 8 erfolgt. Der Grenzbereich hat eine Dicke von mindestens 0,025 mm, bevorzugter von mindestens 0,05 mm, bevorzugter von mindestens 0,1 mm.

Nachdem die Farbpigmente 11 allesamt umhüllt sind, steht die Oberfläche der Farbpigmente 11 selbst nach einer Anpassung des äußeren Durchmessers des ophthalmologischen Implantats nicht/kaum in direktem Kontakt mit Gewebe oder Kammerwasser des Auges, sondern wird insbesondere durch die jeweilige Umhüllung 12 aus biokompatiblem Material vollständig abgeschirmt. Eine unzureichende Biokompatibilität des ophthalmologischen Implantats ist so vermeidbar.

Zur Implantation des ophthalmologischen Implantats wird die Grundstruktur 1 mit ihrer vorderseitigen Lage 7 einer Hornhaut des Auges zugewandt in einem Kapselsack, in einer Hinterkammer oder in einer Vorderkammer des Auges angeordnet. Die Randfläche 5 ist am Sulcus abgestützt. Die Mittelachse 6 verläuft parallel zur optischen Achse einer natürlichen Augenlinse und fällt insbesondere mit dieser zusammen. Zur Fixierung des ophthalmologischen Implantats im Auge wird die Grundstruktur 1 am Kapselsack, am Ziliarkörper, an der Iriswurzel oder an der Sclera, insbesondere mit Nähten aus einem dauerfesten Nahtmaterial, vernäht.

Gemäß einer alternativen Ausführungsform hat die Grundstruktur 1 mehr als zwei Lagen oder genau eine Lage. Alternativ ist die vorderseitige Lage 7 und/oder rückseitige Lage 8 mehrlagig.

## Patentansprüche

1. Ophthalmologisches Implantat, umfassend
a) eine Grundstruktur (1) mit
i) einer zentralen Apertur (2),
ii) einer ersten Seite (3), und
iii) einer der ersten Seite (3) gegenüberliegenden zweiten Seite (4), und
b) eine Vielzahl von in der Grundstruktur (1) angeordneten Pigmentanordnungen (9), wobei mindestens eine der Pigmentanordnungen (9) aufweist
- mindestens ein Farbpigment (11), und
- eine das mindestens eine Farbpigment (11) zumindest größenteils umhüllende Umhüllung (12),
**dadurch gekennzeichnet, dass**
c) jeweils ein Farbpigment (11) von einer eigenen Umhüllung (12) zumindest größtenteils umhüllt ist.

2. Ophthalmologisches Implantat nach Anspruch 1, **dadurch gekennzeichnet, dass** die Umhüllung (12) das mindestens eine Farbpigment (11) der jeweiligen Pigmentanordnung (9) vollständig umhüllt.

3. Ophthalmologisches Implantat nach Anspruch 1 oder 2, **dadurch gekennzeichnet, dass** die Umhüllung (12) eine Umhüllungsaußenform und das mindestens eine Farbpigment (11) der jeweiligen Pigmentanordnung (9) eine Farbpigmentaußenform aufweist, wobei die Umhüllungsaußenform im Wesentlichen der Farbpigmentaußenform des mindestens einen Farbpigments (11) der jeweiligen Pigmentanordnung (9) entspricht.

4. Ophthalmologisches Implantat nach einem der vorherigen Ansprüche, **dadurch gekennzeichnet, dass** die Umhüllung (12) eine Umhüllungsdicke (du) aufweist, die wesentlich kleiner als eine Abmessung des mindestens einen Farbpigments (11) der jeweiligen Pigmentanordnung (9) ist.

5. Ophthalmologisches Implantat nach einem der vorherigen Ansprüche, **dadurch gekennzeichnet, dass** die Umhüllung (12) eine Umhüllungsdicke (du) aufweist, die jeweils zwischen 0,1 µm und 10 µm liegt.

6. Ophthalmologisches Implantat nach einem der vorherigen Ansprüche, **dadurch gekennzeichnet, dass** die Umhüllung (12) biokompatibles Material aufweist.

7. Ophthalmologisches Implantat nach einem der vorherigen Ansprüche, **dadurch gekennzeichnet, dass** die Umhüllung (12), insbesondere außenseitig, reaktive Monomere umfasst.

8. Ophthalmologisches Implantat nach einem der vorherigen Ansprüche, **dadurch gekennzeichnet, dass** die Umhüllung (12), insbesondere außenseitig, Fäden umfasst.

9. Ophthalmologisches Implantat nach einem der vorherigen Ansprüche, **dadurch gekennzeichnet, dass** jede der Pigmentanordnungen (9) einen Umkreisdurchmesser (d_{K}) aufweist, der kleiner als 30 µm, insbesondere kleiner als 10 µm, ist.

10. Ophthalmologisches Implantat nach einem der vorherigen Ansprüche, **dadurch gekennzeichnet, dass** die Pigmentanordnungen (9) in mindestens einer Schicht (10) der Grundstruktur (1) angeordnet sind.

11. Ophthalmologisches Implantat nach einem der vorherigen Ansprüche, **dadurch gekennzeichnet, dass** die Pigmentanordnungen (9) einander zumindest bereichsweise überlappend angeordnet sind.

12. Ophthalmologisches Implantat nach einem der vorherigen Ansprüche, **dadurch gekennzeichnet, dass** die Grundstruktur (1) durch ein Grundstrukturmaterial und die Umhüllung (12) durch ein Umhüllungsmaterial gebildet sind, wobei sich das Grundstrukturmaterial und das Umhüllungsmaterial voneinander unterscheiden.

13. Ophthalmologisches Implantat nach einem der vorherigen Ansprüche, **dadurch gekennzeichnet, dass** das mindestens eine Farbpigment (11) und/oder die dieses zumindest größtenteils umhüllende Umhüllung (12) härter, insbesondere wesentlich härter, als ein Grundstrukturmaterial der Grundstruktur (1) ist.

14. Ophthalmologisches Implantat nach einem der vorherigen Ansprüche, **dadurch gekennzeichnet, dass** jeweils ein Farbpigment (11) von einer eigenen Umhüllung (12) vollständig umhüllt ist.

15. Ophthalmologisches Implantat nach einem der vorherigen Ansprüche, **dadurch gekennzeichnet, dass** die Grundstruktur (1) mehrlagig ist.

16. Verfahren zur Herstellung von Pigmentanordnungen (9) als Bestandteil eines ophthalmologischen Implantats nach einem der vorherigen Ansprüche, umfassend die Schritte:
- Bereitstellen von mindestens einem Farbpigment (11),
- Bereitstellung von Umhüllungsmaterial, insbesondere von biokompatiblem Material, und
- Dispergieren des mindestens einen Farbpigments (11) in dem Umhüllungsmaterial zur Bildung einer Pigmentanordnung (9) mit dem mindestens einen umhüllten Farbpigment (11),
- wobei jeweils ein Farbpigment (11) von einer eigenen Umhüllung (12) zumindest größtenteils umhüllt ist.

17. Verfahren nach Anspruch 16, **gekennzeichnet durch** eine Vielzahl von Farbpigmenten (11), wobei die Konzentration der Farbpigmente (11) in dem biokompatiblen Material erhöht wird.

## Claims

1. Ophthalmological implant, comprising
a) a main structure (1) with
i) a central aperture (2),
ii) a first side (3); and
iii) a second side (4) arranged opposite the first side (3); and
b) a plurality of pigment arrangements (9) arranged in the main structure (1), wherein at least one of the pigment arrangements (9) has
- at least one color pigment (11); and
- an enclosure (12) enclosing at least most of the at least one color pigment (11),
**characterized in that**
c) at least most of one color pigment (11) at a time is enclosed by its own enclosure (12).

2. Ophthalmological implant as claimed in claim 1, **characterized in that** the enclosure (12) completely encloses the at least one color pigment (11) of the respective pigment arrangement (9).

3. Ophthalmological implant as claimed in claim 1 or 2, **characterized in that** the enclosure (12) has an enclosure outer shape and the at least one color pigment (11) of the respective pigment arrangement (9) has a color pigment outer shape, wherein the enclosure outer shape substantially corresponds to the color pigment outer shape of the at least one color pigment (11) of the respective pigment arrangement (9).

4. Ophthalmological implant as claimed in any one of the preceding claims, **characterized in that** the enclosure (12) has an enclosure thickness (du), which is much smaller than a dimension of the at least one color pigment (11) of the respective pigment arrangement (9).

5. Ophthalmological implant as claimed in any one of the preceding claims, **characterized in that** the enclosure (12) has an enclosure thickness (d_{U}), which is between 0.1 µm and 10 µm in each case.

6. Ophthalmological implant as claimed in any one of the preceding claims, **characterized in that** the enclosure (12) has biocompatible material.

7. Ophthalmological implant as claimed in any one of the preceding claims, **characterized in that** the enclosure (12) comprises reactive monomers in particular on an outside thereof.

8. Ophthalmological implant as claimed in any one of the preceding claims, **characterized in that** the enclosure (12) comprises fibers, in particular on an outside thereof.

9. Ophthalmological implant as claimed in any one of the preceding claims, **characterized in that** each of the pigment arrangements (9) has a circumferential diameter (d_{K}), which is smaller than 30 µm, in particular smaller than 10 µm.

10. Ophthalmological implant as claimed in any one of the preceding claims, **characterized in that** the pigment arrangements (9) are arranged in at least one layer (10) of the main structure (1).

11. Ophthalmological implant as claimed in any one of the preceding claims, **characterized in that** the pigment arrangements (9) are arranged such as to overlap each other at least partly.

12. Ophthalmological implant as claimed in any one of the preceding claims, **characterized in that** the main structure (1) is formed by a main structure material and the enclosure (12) is formed by an enclosure material, wherein the main structure material and the enclosure material differ from one another.

13. Ophthalmological implant as claimed in any one of the preceding claims, **characterized in that** the at least one color pigment (11) and/or the enclosure (12) enclosing at least most of said color pigment (11) is harder, in particular much harder, than a main structure material of the main structure (1).

14. Ophthalmological implant as claimed in any one of the preceding claims, **characterized in that** the entirety of one color pigment (11) at a time is enclosed by its own enclosure (12).

15. Ophthalmological implant as claimed in any one of the preceding claims, **characterized in that** the main structure (1) has a multi-ply design.

16. Method for producing pigment arrangements (9) as a component of an ophthalmological implant as claimed in any one of the preceding claims, comprising the steps:
- providing at least one color pigment (11);
- providing enclosure material, in particular of biocompatible material; and
- dispersing the at least one color pigment (11) in the enclosure material to form a pigment arrangement (9) with the at least one enclosed color pigment (11),
- wherein at least most of one color pigment (11) at a time is enclosed by its own enclosure (12).

17. Method as claimed in claim 16, **characterized by** a plurality of color pigments (11), wherein the concentration of the color pigments (11) in the biocompatible material is being increased.

## Revendications

1. Implant ophtalmique, comprenant
a) une structure de base (1) comportant
i) une ouverture centrale (2),
ii) un premier côté (3), et
iii) un second côté (4) opposé au premier côté (3), et
b) une pluralité d'ensembles pigmentaires (9) disposés dans la structure de base (1), au moins l'un des ensembles pigmentaires (9) comportant
- au moins un pigment coloré (11), et
- une enveloppe (12) enveloppant au moins en grande partie l'au moins un pigment coloré (11),
**caractérisé en ce**
c) **qu'**un pigment coloré (11) est enveloppé au moins en grande partie par sa propre enveloppe (12).

2. Implant ophtalmique selon la revendication 1, **caractérisé en ce que** l'enveloppe (12) enveloppe entièrement l'au moins un pigment coloré (11) de l'ensemble pigmentaire (9) respectif.

3. Implant ophtalmique selon la revendication 1 ou 2, **caractérisé en ce que** l'enveloppe (12) présente une forme d'enveloppe extérieure et l'au moins un pigment coloré (11) de l'ensemble pigmentaire (9) respectif présente une forme extérieure de pigment coloré, la forme d'enveloppe extérieure correspondant sensiblement à la forme extérieure de pigment coloré de l'au moins un pigment coloré (11) de l'ensemble pigmentaire (9) respectif.

4. Implant ophtalmique selon l'une des revendications précédentes, **caractérisé en ce que** l'enveloppe (12) a une épaisseur d'enveloppe (du) sensiblement inférieure à une dimension de l'au moins un pigment coloré (11) de l'ensemble pigmentaire (9) respectif.

5. Implant ophtalmique selon l'une des revendications précédentes, **caractérisé en ce que** l'enveloppe (12) a une épaisseur d'enveloppe (du) comprise entre 0,1 et 10 µm.

6. Implant ophtalmique selon l'une des revendications précédentes, **caractérisé en ce que** l'enveloppe (12) renferme un matériau biocompatible.

7. Implant ophtalmique selon l'une des revendications précédentes, **caractérisé en ce que** l'enveloppe (12) comprend des monomères réactifs, en particulier sur le côté extérieur.

8. Implant ophtalmique selon l'une des revendications précédentes, **caractérisé en ce que** l'enveloppe (12) des fils, en particulier sur le côté extérieur.

9. Implant ophtalmique selon l'une des revendications précédentes, **caractérisé en ce que** chacun des ensembles pigmentaires (9) a un diamètre circonférentiel (d_{K}) inférieur à 30 µm, en particulier inférieur à 10 µm.

10. Implant ophtalmique selon l'une des revendications précédentes, **caractérisé en ce que** les ensembles pigmentaires (9) sont disposés dans au moins une couche (10) de la structure de base (1).

11. Implant ophtalmique selon l'une des revendications précédentes, **caractérisé en ce que** les ensembles pigmentaires (9) sont disposés de manière à se chevaucher au moins par endroits.

12. Implant ophtalmique selon l'une des revendications précédentes, **caractérisé en ce que** la structure de base (1) est constituée d'un matériau de structure de base et l'enveloppe (12) est constituée d'un matériau d'enveloppe, le matériau de structure de base et le matériau d'enveloppe étant différents l'un de l'autre.

13. Implant ophtalmique selon l'une des revendications précédentes, **caractérisé en ce que** l'au moins un pigment coloré (11) et/ou l'enveloppe (12) l'enveloppant au moins en grande partie est plus dur, en particulier sensiblement plus dur, qu'un matériau de structure de base de la structure de base (1).

14. Implant ophtalmique selon l'une des revendications précédentes, **caractérisé en ce qu'**un pigment coloré (11) est entièrement enveloppé par sa propre enveloppe (12).

15. Implant ophtalmique selon l'une des revendications précédentes, **caractérisé en ce que** la structure de base (1) est multicouche.

16. Procédé de fabrication d'ensembles pigmentaires (9) comme constituant d'un implant ophtalmique selon l'une des revendications précédentes, comprenant les étapes :
- de fourniture d'au moins un pigment coloré (11),
- de fourniture de matériau d'enveloppe, en particulier de matériau biocompatible, et
- de dispersion de l'au moins un pigment coloré (11) dans le matériau d'enveloppe pour former un ensemble pigmentaire (9) dans lequel l'au moins un pigment coloré (11) est enveloppé,
- un pigment coloré (11) étant enveloppé au moins en grande partie par sa propre enveloppe (12).

17. Procédé selon la revendication 16, **caractérisé par** une pluralité de pigments colorés (11), la concentration des pigments colorés (11) dans le matériau biocompatible étant accrue.
